# EUROPEAN PATENT SPECIFICATION

(11) **EP 1 596 924 B1**
(45) Date of publication and mention of the grant of the patent: **30.03.2011**
(21) Application number: 04708801.8
(22) Date of filing: 06.02.2004
(51) Int. Cl.: A61M 35/00, B05B 12/12, B05B 15/12

(54) **TANNING BOOTH**
BRÄUNUNGSKABINE
CABINE DE BRONZAGE

(30) Priority: 07.02.2003 GB 0302853; 21.03.2003 GB 0306553; 10.10.2003 GB 0323794
(43) Date of publication of application: 23.11.2005
(73) Proprietor: Beauty Source Ltd., Chilwell Nottingham NG9 6QG (GB)
(72) Inventor: SMITH, Warrick, J., Worthing, West Sussex BN14 7PS (GB); STAFFORD-NELSON, Christopher, Coventry CV3 6BY (GB)
(74) Representative: Johnstone, Helen Margaret
(86) International application number: PCT/GB2004/000478
(87) International publication number: WO 2004/069322

(56) References cited:
- EP-A- 0 313 525
- EP-A- 1 238 642
- WO-A-00/54892
- WO-A-00/62640
- US-A- 3 594 211
- US-A- 5 418 985

## Description

This invention relates to a booth in which a person may have a product such as a cosmetic product applied to her skin. The invention relates particularly to a tanning booth in which a person positioned within the booth may have a sunless tanning lotion applied to her skin.

Recently, public awareness of the harmful side effects of the sun's rays has become higher. It is well known that too much exposure to the sun can lead to skin cancer. Because of this risk, the popularity of cosmetic products which provide a "sunless" tan has increased significantly over recent years.

Existing sunless tanning lotions are often in the form of creams or lotions that may be self applied to the skin. A problem with such creams and lotions is that it is very difficult to ensure an even tan since the tan often is not visible until sometime after the cream or lotion has been applied to the skin.

It is also known to have such creams and products applied by a beautician. An advantage of this method is that a more even tan is usually achieved.

More recently it has been known to apply a sunless tanning lotion to the skin of a person with an air brush. This known method involves a beautician or other operator applying a sunless tanning lotion to a client using an air brush to spray the sunless tanning product over the skin of the client to achieve an even coverage over the skin.

A problem with this existing method is that although tanning products are not harmful when inhaled by beauticians applying the product to clients, beauticians may be exposed over significant periods of time to particles of sunless tanning lotion which may "hang" in the atmosphere during and after the air brushing process. In addition, a client to which the product is applied is often surrounded by a mist of the tanning product during and after the spraying process and so may inhale a significant quantity of the product during the tanning process. This can cause discomfort.

EP 1238 642 describes an apparatus for the coating of a human body with a tanning composition such as a sunless tanning composition. The discloses apparatus comprises an arm with a plurality of nozzles thereon which traverses within a booth to spray coat a body in the booth. The spray nozzles are orientated to avoid opposing airflows and excessive air flows which cause inefficient and uneven dripping or streaking in the deposition of tanning composition on the skin.

A tanning booth is described in our co-pending International patent application of even date.

According to a first aspect of the present invention there is provided a booth for accommodating a person, the booth defining a booth volume and comprising:
a base portion and a top portion;
flow means for causing a downward airflow within the booth;
projecting means for projecting a sunless tanning product into at least some of the booth volume within the booth and, in use, onto a body of a person positioned in the booth; and
control means for controlling the projection of the product, wherein the control means further comprises a sensor for sensing the height of the person, and estimating means for estimating one or more other parameters of the body based on the height, the control means controlling the amount of product directed to predetermined parts of the body in response to the height of the person.

The booth is adapted to accommodate a person requiring a product to be applied to her skin. The downward air flow within the booth means that the product projected within the booth and therefore onto the body of a person positioned within the booth is carried downwards in the air flow. This means that fewer particles of the product remain in the atmosphere and therefore fewer particles will be inhaled by the person receiving the tan.

Because the booth according to the first aspect of the invention comprises control means for controlling the projection of the product, it is not necessary for an operator to administer the product or control the projection means. This reduces, or eliminates an operator's exposure to the product.

The control means comprises a sensor for sensing the height of the body.

The control means controls the projection of the product according to the height of the body of the person positioned in the booth.

People using a booth according to the invention will fall within a range of heights. In general, therefore, the booth of the invention is adapted to accommodate a body having a height of up to 1.94m (6'4") and down to a minimum of 0.85 m. Theoretically there is not a minimum height, although practicably one cannot spray a person if her height is zero. Preferably, however a minimum height of 850mm is created in order that if the sensor has not picked up anything by this height, it indicates to the system that there has been a malfunction in the sensing operation and therefore causes the system to reset

In prior art booths, the amount of product projected onto a body within the booth would be the same regardless of the height of the body standing in the booth at any given time. This means that, in order to ensure that sufficient product is projected onto a person of maximum height, all people using the booth will have a maximum amount of product projected onto them. Because prior art booths are designed to cater for the largest body size which can be handled, consumption of the product is correspondingly unnecessarily high.

By means of the present invention, because the projection of the product is controlled in accordance with the height of the body in the booth, product consumption can be tailored to suit each person's height thus reducing or eliminating wastage of product on shorter clients.

Although all human bodies are broadly of the similar shape, each body displays a set of unique nuances in shape and size. As mentioned herein above, this can cause problems with the automating spraying or projecting of a product onto a human body.

By means of the sensor therefore, relevant data from a body accommodated in the booth can be gathered. The data can then be used by the control means to vary the projection of the product to accommodate features of the body accommodated within the booth.

The projection of the product can be varied in accordance with the exact size and shape of each human body accommodated in the booth. However, a control means adapted to respond in this way to each different body accommodated in the booth would be time consuming to develop and therefore expensive.

The inventors have realised that in respect of the present invention, the most important parameter of a human body is the height of that body. Little if any further parameters are required in order to obtain sufficient data for the control means to effectively control the projection of the product. Other features of the body can be deduced by calculating the position of these features based on a percentage of the body's height. These percentages are based on mean averages of data taken from a large number of bodies.

For example; by means of the present invention it is possible to produce an approximate location of a client's ears. It is generally a problem with known booths that the rear of a client's ears is unnecessarily coated with product, causing inconvenience and a lack of realism in the resultant tan.

By means of the present invention, it is possible to prevent product from being sprayed onto the back of a client's ears thus resulting a more realistic tan.

The sensor may be any suitable type of sensor, but preferably the sensor comprises an infra-red sensor.

Advantageously, the booth further comprises movement means for effecting movement of the projecting means.

Preferably, operation of the projection means is controlled by the control means.

Advantageously, the movement means effects vertical movement of the projecting means. This means that the height from which the product is projected may be varied both throughout the tanning operation, and also in response to the parameters of the body accommodated in the booth.

Advantageously, the movement means comprises at least one slider unit moveable vertically between two positions, the slider unit supporting the projecting means.

Preferably, the projecting means comprises at least one nozzle adapted to project the product.

Preferably, the projecting means comprises a plurality of nozzles each adapted to project the product. Through use of a plurality of nozzles, the product may be projected into at least some of the booth volume, and, in use onto a body of a person from a plurality of different projection points.

Advantageously, the booth further comprises adjustment means for adjusting the attitude of the nozzles. The adjustment means may be adapted to either adjust the attitude of the nozzles independently from one another, or to adjust the attitude of the nozzles so that all nozzles have the same attitude.

Advantageously, operation of the adjustment means is controlled by the control means.

Preferably, the projection means comprises a nozzle support defining a substantially arcuate shape, the nozzle being positioned to spray the product into an area defined by the nozzle support.

By means of the arcuate nozzle support, the product may be projected towards the centre of the booth into an area, where in use, a person will be accommodated.

The nozzle support may define a substantially circular shape or alternatively may define a semi-circular or other part-circular shape.

Preferably, the projecting means comprises one or more spray guns which are directed to spray product horizontally and/or at an angle to the horizontal (whether upwardly or downwardly, and/or some combination of these).

Alternatively, there is provided one or more slider units supporting an arcuate spray arm comprising a plurality of spray guns.

Advantageously, the booth further comprises a fluid delivery system for delivering the product into the booth via the projecting means.

Preferably, the fluid delivery system forms part of a recirculating fluid circuit which further includes the projecting means. The projecting means are thus able to be fed with fluid within the fluid delivery system. Any excess fluid that is not dispensed by the projecting means is returned to the fluid delivery system.

Advantageously, fluid is contained within a fluid container, and product which is not dispensed by the projecting means is returned by means of one or more pipes to the fluid container.

In order to project the product as a spray, the fluid delivery system delivers the product in liquid form, and also delivers air to the projection means for mixing with the product to form the spray.

Conveniently, the fluid delivery system delivers air to the projection means at a predetermined air pressure. Advantageously, the air pressure is varied. The air pressure is one of the factors that will contribute to the quality of the resulting tan.

Preferably, the fluid delivery system delivers product to the projection means at a fluid pressure.

Advantageously, the booth further comprises pressure means for controlling the fluid pressure.

Preferably, the pressure means comprises a fluid pump.

Conveniently, the fluid delivery system delivers product in form of a fluid to the projection means at a predetermined pressure.

Advantageously, the fluid pressure is varied. The fluid pressure will govern how much product is dispensed per second into the booth.

The pressure within the fluid delivery system is controlled by controlling how much resistance there is to movement of the fluid back into the container from which is it drawn - i.e. controlling the backpressure within the fluid circuit.

When the projecting means are operating at a particular setting, they will allow more or less product to be dispensed in direct relation to the pressure of the fluid that is feeding them. Less pressure in the fluid delivery circuit means that at the particular setting, the projecting means will dispense less fluid per second than if the fluid pressure is increased within the fluid delivery system.

Air and product are mixed together within the fluid delivery system to produce an atomising air stream so that the product is projected from the projecting means in an atomised form contained within an air stream. Preferably the projecting means comprises a gun. The gun may create a conical spray pattern when projecting product into the booth, but preferably, the gun creates a pattern which is wider in one plane than another. Such a gun is known as a fan gun.

Advantageously, the spray pattern is wide in a substantially horizontal plane, and narrow in a substantially vertical plane.

The pressure at which the atomising air stream exits the projecting means, which is known as the atomising air pressure, causes differences in the way that the product is sprayed into the booth. If the atomising air pressure is raised, the product will be spread into a wider spray pattern in the horizontal plane, will project further, and the product will be atomised into finer particles.

When the product comprises a sunless tanning product, the depth of the tan may be controlled by controlling the pressure of the product in the fluid delivery system.

By controlling the pressure within the fluid delivery system to the projecting means, together with the speed at which the projection means move, the amount of the product dispensed per second, and the distribution of the product dispensed can be controlled.

Further, by controlling the air speed of the downward air flow within the booth, further control of fluid projection may be achieved.

Preferably, the booth further comprises recirculating means for recirculating the air within the booth. The recirculating means comprises any suitable air moving device such as a fan.

Advantageously, the booth further comprises a filter. The filter filters out particles of the product circulating within the booth.

Preferably, the booth comprises a plurality of filters forming a filtration system.

The booth may be used to apply a cosmetic product to the skin of the user. The cosmetic product could be any of a range of products such as skin moisturisers, but preferably the cosmetic product is a sunless tanning product.

Advantageously, the flow means comprises a first plenum of positive pressure located in the top portion of the booth, and a second plenum of negative pressure that is located in the base portion of the booth. The first and second plenums therefore result in the downward flow of air from the first plenum towards the second plenum.

The downward airflow may be arranged to occupy a predetermined volume within the booth. A person occupying the booth may stand in this predetermined volume during and after the tanning process.

Advantageously, the predetermined volume comprises a portion only of the booth volume.

This means that, for example, a client may leave personal belongings in a portion of the booth situated outside of the predetermined volume, which belongings will therefore not be exposed to the particles of sunless tanning lotion.

The recirculating means is used to maintain the pressure differential between the first and second plenums and therefore contributes to the down flow of air within the booth.

Conveniently the first plenum pressurises air in excess of the ambient atmospheric pressure and the second plenum depressurises air to less than the ambient atmospheric pressure.

Advantageously the first and second plenums are connected to a duct in which the air recirculating means is located. The first and second plenums, the duct, the recirculating means and the booth volume together form an air management system.

Preferably the booth further comprises temperature means for controlling the temperature of air circulating within the booth. The temperature means may comprise a heater which heats the air or alternatively may comprise a cooler which cools the air.

The temperature means may maintain the air within the booth at approximately 29°C to 30C. Preferably, however, the temperature means maintains the air within the booth at 33°C.

Preferably, the control means operates the projecting means to project specified amounts of the product in selected regions of the booth volume, the specified amounts varying from zero to maximum flow of the product from the projecting means.

Preferably, the projecting means is movable along a path within the booth and the control means operates the projecting means as it moves along the path in accordance with predetermined instructions.

There is also provided a computer program product directly loadable into the internal memory of a digital computer, comprising software code portions for performing the steps of the method when said product is run on a computer.

Preferably, the projecting means comprises a remotely operated tool.

According to a further aspect of the present invention there is provided a tool for projecting a product into a booth, the booth comprising a base portion and a top portion, flow means for causing a downward air flow within the booth, and control means for controlling the projection of the product.

In a preferred embodiment of the present invention, the projecting means comprises movement means to automatically move the tool to provide spraying between two zones in the booth. Preferably, the two zones comprise substantially the entire height of the booth.

The spray guns may be directed to spray product horizontally and/or at an angle to the horizontal (whether upwardly or downwardly) and/or some combination of these.

According to a further aspect of the present invention there is provided a method of applying a product to a human body using a booth, the booth defining a booth volume and comprising:
a base portion and a top portion;
flow means for causing a downward air flow within the booth; and
projecting means for projecting a product into at least some of the booth volume within the booth and, in use, onto a body of a person positioned in the booth, and control means for controlling the projection of the product.
   the method comprising the steps of:
sensing the height of a human body accommodated in the booth;
estimating one or more other parameters of the body based on the height;
projecting a product into the booth and therefore onto the body;
controlling the projection of product into predetermined zones in the booth in accordance with the height of the body.

Preferably the step of projecting a product into the booth comprises spraying the product via the projecting means into the booth.

Advantageously, the booth comprises a fluid delivery system for delivering air and product to the projection means.

Preferably, the step of controlling the projection of product into predetermined zones within the booth in accordance with the height of the body, comprises one or more of the following steps:
controlling the pressure at which product is delivered to the projecting means;
controlling the pressure at which air is delivered to the projecting means.

Preferably, the step of controlling the projection of product projected into predetermined zones further comprises the step of causing the projecting means to move vertically within the booth, and controlling the speed at which the projecting means move.

Advantageously, the step of controlling the projection of the product further comprises the step of controlling the air speed of the downward air flow within the booth.

By means of the present invention, it is possible to direct the product to a predetermined point within the booth, and therefore to a predetermined part of a body standing in the booth. It is also possible to calculate accurately the amount of product required for different parts of the body.

By means of the present invention, therefore, because the control means is able to sense the height of a body standing in the booth, and then to determine where other parts of the body are located relative to the base of the booth, the amount of product projected by the projecting means can be varied in order to ensure that appropriate amounts of product are directed to predetermined parts of the body, in an appropriate manner.

By means of the present invention, therefore, the way in which a product is applied to a person within a booth according to the present invention involves control of some or all of the following parameters:
air pressure to the projecting means;
atomising air pressure to the projecting means;
speed of movement of the projecting means within the booth;
fluid pressure to the projecting means; and
air speed of the downward air flow.

Although the present invention has been described in terms of a booth, it would be possible to carry out the invention in an open environment. Similarly, it would be possible to carry out the invention within an area defined by the downward flow of air, which downward flow of air would ensure containment of the product within a predetermined volume.

The invention will now be further described by way of example only with reference to the accompanying drawings in which;
Figure 1 is a schematic representation of a booth according to a first aspect of the present invention;
Figure 2 is cross-sectional representation of the booth of Figure 1;
Figure 3a is a schematic representation of the booth of Figure 1 showing product being projected onto a person standing in the booth having a predetermined fluid projection distance;
Figure 3b & Figure 3c are schematic representations showing product being projected onto the person in the booth of Figure 1 having increasing fluid projection distances;

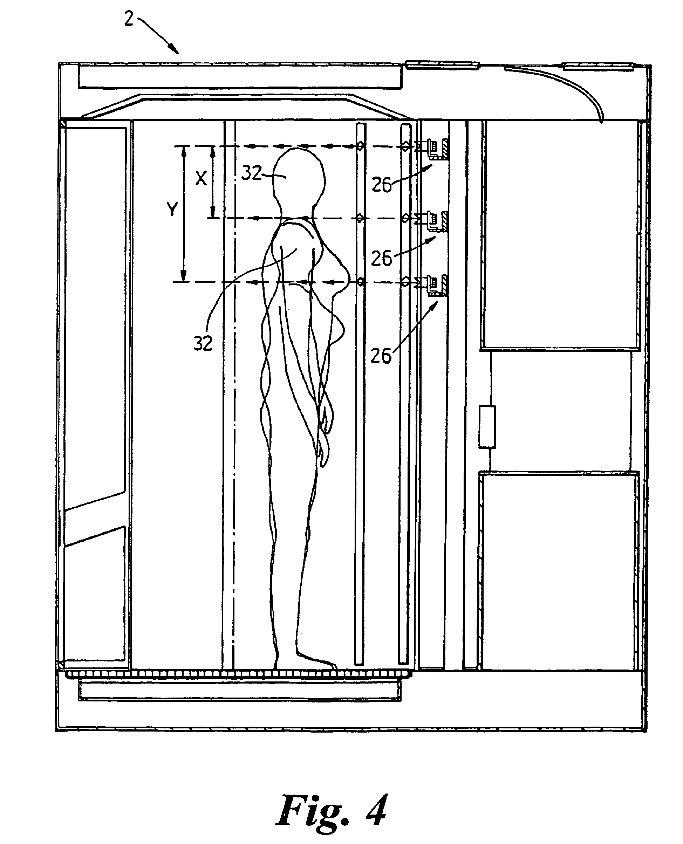

Figure 4 is a schematic representation showing how bodies of different heights may be accommodated within the booth;
Figure 5 is a schematic representation showing how a predetermined amount of product may be projected into a predetermined zone of the booth, thus, in use, applying more product to certain parts of the body in the booth;
Figure 6a and 6b are schematic representations showing the different positions taken by a body within the booth;
Figure 7 is a perspective part view of a slider unit forming part of the booth of Figure 1;
Figure 8 is a schematic representation showing in detail how a booth according to the invention would be operated; and
Figure 9 is a block diagram showing the components of a booth according to the present invention; and
Figure 10 is a flow chart of one way of applying product to a person using a booth according to the invention.

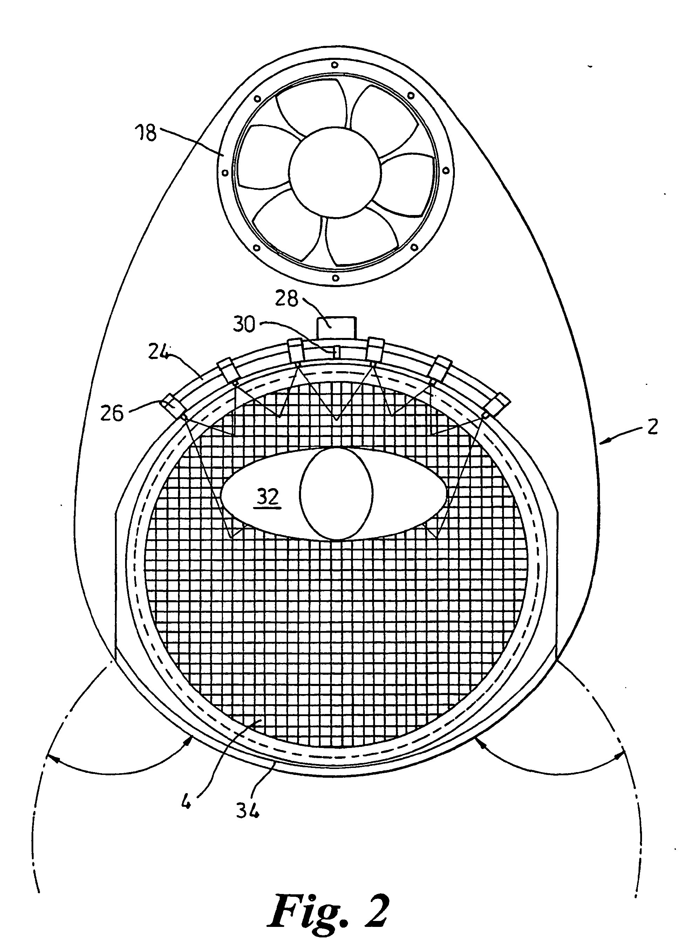

Referring to the figures, a booth according to the first aspect of the present invention is designated generally by the reference numeral 2. The booth defines a booth volume also known as a spray chamber 4 and comprises a base portion 6 and top portion 8. The booth 2 comprises an upper plenum 10, and a lower plenum 12. The upper plenum has a positive pressure with respect to atmospheric pressure, and the lower plenum has a negative pressure with respect to atmospheric pressure. This pressure difference causes a downward flow of air, in the direction of arrows 14, of air circulating within the booth 2. The booth 2 further comprises a duct 16 comprising an air mover 18 in the form of, for example, a fan (see Figure 2). The air mover 18 positioned in the duct 16 allows air to be recycled within the booth 2. The booth further comprises a filtration system 20, the purpose of which will be described further herein below.

The booth 2 further comprises projection means 22 comprising a spray ring 24 on which are mounted a plurality of spray guns, or nozzles 26. The spray ring 24 is mounted on a slide carriage 28 which allows vertical movement of the spray ring. The booth 2 further comprises a range sensor 30 mounted on the spray ring 24. The range sensor is used to detect the height of a person or client 32 standing the booth as will be described in more detail herein below.

The booth further comprises doors 34 which are moveable between a sealed positioned as shown by the solid lines in Figure 2, and an open position as shown by the broken lines in Figure 2.

The spray ring 24 on which the spray guns 26 are mounted, enables the guns 26 to be mounted in precise predetermined positions.

The spray ring is in turn mounted on the slide carriage 28. The slide carriage 28 enables the spray ring to reciprocate from the top of the spray chamber 4 to the bottom of the spray chamber. This enables the guns 26 mounted on the spray ring to be moveable along a predetermined distance, for example, along the entire length of a client 32 having a maximum height of, for example, 6' 4". The slide carriage 28 is caused to reciprocate by way of an electrical motor (not shown) and is driven via any suitable drive shaft mechanism.

The spray guns deliver product into the spray chamber and cause the product to be atomised on entry into the spray chamber.

During the act of spraying, the tanning product becomes air borne in the form of small droplets. The downward airflow of the present invention ensures that these small droplets are kept away from the client's face and that a clean supply of air for the client is available. The filtration system 20 serves to filter out particles of the product to ensure that the recirculating air is as clean as possible.

In other words, although a client's face is sprayed with product, the face is exposed to excess product only from the time that the projecting means are above the face, or pointing directly on it. Due to the height sensor forming part of the present invention, this time period is approximately the same for each client, thus creating a safer system from the inhalation point of view.

Typically, the time that a client will have excess spray particulate in the vicinity of their face will not exceed approximately 2 seconds for each pass of the slide carriage. In addition, because of the downward air flow within the booth, any excess product is drawn away from a client's face. Further, when the rear of a person is being sprayed, in order to avoid excess coating of the rear of the ears of a client, the projection means begins spraying at a point which is lower in height than the corresponding point when the front of the body of the client is sprayed. This means that exposure in the head area of a client when the rear of their body is being sprayed is typically less than 1 second.

A percentage of the air flow is bled off from the first plenum 10 in order that the differential between the air volume being drawn from the booth volume 4 into the second plenum and the air volume being moved into the booth volume 4 from the first plenum 10 is replaced by air movement into the downward flow of air within the booth volume. This management of the air flow prevents any outward flow of particles from the booth volume 4 during the spraying process and ensures that all particles generated within the booth volume 4 must pass through the filtration system 20 thus ensuring that the client is maintained in clean air.

The filtration system 20 filters substantially all wet and dry particulate material from the recirculating air.

A downward flow of air is the most efficient way to move air over a human body as moving air downwards over a body ensures that the body presents a minimum cross sectional area of impedance to the air flow.

The downward air flow capitalises on the tendency for heavy atomised particles to drop downwards under the influence of gravity. The downward airflow thus uses gravity to assist in the process of extracting particles generated by the spraying tool from the booth volume 4.

Since air is recycled within the booth 2, it will not be necessary for a purchaser of the booth 2 to carry out building work to their premises in order to create ductwork to the atmosphere. By using a recirculating air stream there is no need for any building work to be carried out since no ductwork to the atmosphere is required.

The booth 2 further comprises a heater 34 which warms the air in the downward airflow. The combination of spraying a wet product onto a client's skin in conjunction with an air stream moving over the body of a client creates a "chill factor". By means of the heater 26 it is possible to ensure that air passing over the body of a client is at an appropriate temperature. Further, after the spraying process has been completed, a client may be dried by the airflow.

Although the invention as hitherto been described with reference to a heating apparatus for heating the air flowing through the booth, it is envisaged that in warmer climates or during the summer of temperate climates it may be advantageous to cool the air flow.

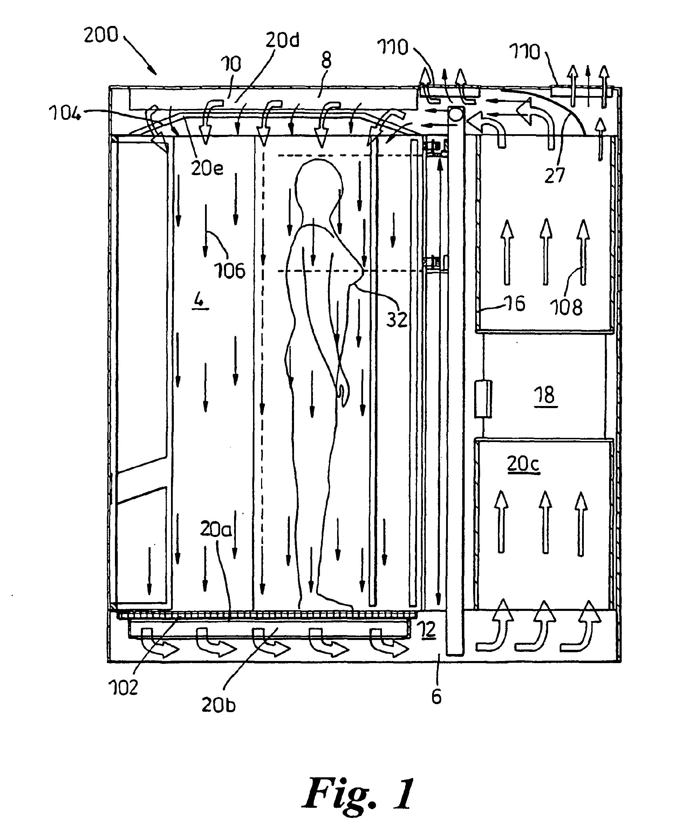

The fan 18 causes the air within the recirculated down draught extract system (indicated by arrows 14 in Figure 1) to be moved within the booth. The speed at which the fan is caused to run influences how much air volume is moved through the fixed volume of the air management system, therefore influencing the speed of the air moving downward within the spray chamber.

The range sensor 30 is able to detect the position of features on the client's body such as the top of the head of the client's body. The sensor works by "seeing" only within a certain range. In the present embodiment the range of the sensor is 700mm. It will sense anything within this range. At this range the sensor will sense only what is actually inside the spray chamber and not, for example, the inside of the doors should they be closed. A client stands within the range of the sensor when inside the spray chamber thus enabling the range sensor to detect the top of the client's head.

The booth 2 further comprises filtered constant bleed sites 36 for ensuring that the pressure does not build up within the booth. These bleed sites ensure that the pressure differentials within the two plenums obey the following rule in order to ensure that there is no possibility of atomised product leaving the spray chamber other than through the filtration system:
Overpressure in upper chamber-one atmosphere = X
Underpressure in lower chamber- one atmosphere = Y

With the effect of the bleed sites, Y is always greater than X thus causing an IN-flow of air through any leak sites within the spray chamber itself, thus ensuring that no atomised product can leave the spray chamber other than via the filter systems.

The booth 2 further comprises a control system 90 which can take any suitable form, but in this embodiment comprises electrical and electronic components which are run by real time embedded software. The control system controls the general operation of the booth 2, and all of the variable parameters within the system. The control system is able to self-teach. This means that once the sensor has sensed the height of a person standing in the booth 2, the control system is able to work out the necessary parameters for controlling projection of product into the booth 2. The control system is able to work out how to appropriately control the projection of product into the booth by initially working out where different parts of the body are located relative to the base of the booth, based on the known proportions of an average human body. The control system is then able to work out variables such as the speed of the fan, speed of movement of the spray ring along the slide carriage 28, and timings for switching on and off the spray guns in order to apply product appropriately to the person standing in the booth. The control system is also able to calculate the points at which the air and fluid pressure are varied to the guns, and the points at which the fan speed alters, where the gun carriage accelerates and slows down, as well as where it moves from and to. For each cycle of the booth, these parameters are calculated again, and in this embodiment are not stored in the memory within the control system.

In some embodiments of the invention, it is also possible to increase or decrease the fluid pressure to the guns. This results in a client being given a darker or lighter tan depending on the fluid pressure to the guns.

The type of tan required could be selected by a particular client, each time that client enters the booth. However, in certain embodiments of the invention, in order to cut down on the information that a client has to input into the booth on entry to the booth, information relating to the preferences of a particular client could be stored on magnetic media, such as a credit card type strip. This means that a client may simply walk into a booth and swipe their settings into the booth, so that they generate their last profile parameter set which includes the preferred depth of tan setting in the most convenient way possible.

The particular settings calculated by the control system 90 are known as a profile parameter set.

In use, a person requiring product such as a self-tanning product to be applied to her skin, enters the booth 2. Once the client 32 is within the booth, the doors 34 are closed in order to create a sealed environment. The client 32 is instructed, for the sake of simplicity, to place her feet on fixed plates 36 located on the floor of the booth 2. This ensures that the client is standing in a repeatable position during the operation of the booth. Once the client is in position, the range sensor 30 senses the height of the top of the client's head. Once the height of the top of the client's head has been sensed, all other relevant points on the client's body can be deduced with sufficient precision by calculating the position of these parts of the body as percentages of the client's height. These percentages are based on mean averages of data taken from a large number of individuals. The sensor 30 detects the height of the client by reciprocating from the top of the spray chamber towards the bottom along the slide carriage 28. It is not necessary for the sensor to reciprocate along the entire length inside the spray chamber. It is necessary only for the sensor to reciprocate downward until a signal is emitted from the sensor when it determines the presence of the top of the client's head. Once this information has been gathered, the slide carriage 28 returns the spray ring to the position which is appropriate to start the profile parameter set for which it has just gathered the information. All profile parameter sets start at a fixed dimension over the client's head. For example should the client be 1700mm tall then the slide will proceed downward from the "home" position in which if rests (the uppermost reach of its stroke) until the signal is gained from the sensor i.e. at 1700mm. It will then slow down and stop in a controlled manner, whereupon it will reciprocate upwards to the first point in the profile parameter set created for that client. The profile parameter sets start the spraying operation 50mm above the top of the client's head. Therefore in this case, the slide would return upward to a point which places the gun nozzles at a height of 1750mm. This is the point at which this particular profile parameter set would cause the application of product to be started. However, if the sensor or sensors are used in a more detailed manner i.e. to sense further information regarding the client's body, the slide carriage 28 may be required to reciprocate the slide all the way to the bottom of the spray chamber.

Once the height of the client has been determined, the profile parameter set is created. This is a set of instructions issued by the control system to the components of the spray booth system in order to achieve application of the product as dictated by the information gathered and processed. Once the profile parameter sets have been created, the projection means are able to project product into predetermined zones within the booth so that the application of product onto the client is optimised.

Other information which is deducible from the height of the client is the approximate position of the upper and lower body, divided at the hip line. This enables the system to be able to carry out partial body tans correctly sized to the client's body. Clearly a 6' tall person would have a hip line in a very different position relative to the base of the booth than a 5' tall person. By means of the present invention it is possible to offer partial body tans, either upper or lower, to clients since it is possible to determine where the hip line of a particular client is relative to the base of the booth.

The depth of the tan produced by application of the product to the person standing the booth can be controlled by controlling the pressure that the tanning product is delivered to the projection means via the fluid delivery system.

It is therefore, possible for a client to select a "tan depth".

The applicant has become aware that different skin types absorb product differently.

Generally speaking, fair skin types will absorb product more slowly, and to a lesser extent in any one application.

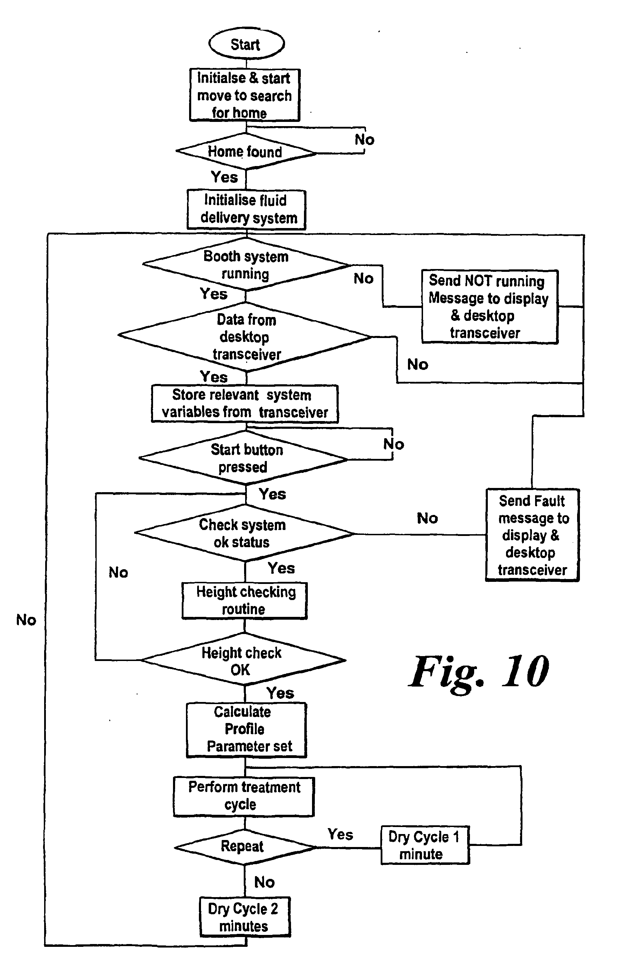

A preferred treatment cycle therefore, comprises two cycles of treatment during which 55% of the normal dosage of product is delivered to a person standing in the booth, each cycle being punctuated by a one minute pause. The pause allows the skin to absorb the product. This absorption process is assisted by the downward air flow within the booth which is generally heated to a temperature of approximately 29 - 30°C. After the second cycle, there is two minute drying period. A flow chart showing such a cycle is illustrated in Figure 10.

For all skin types, a very dark tan can be achieved using a twin cycle, with up to 100% of the normal dosage being delivered in each cycle.

However, it has been found that it is preferable to deliver 75% of the normal dosage in each of the two cycles. This will result in a client having 150% of the normal dose applied to her body. However, by applying the product in two cycles, the skin is allowed to absorb the product between the two applications. This prevents wetting of the product on the skin of a client, and therefore also prevents potential streaking.

Of course it falls within the scope of the present application, that different types of cycles may be used to administer product to a particular person.

The control system controls the speed of the slide carriage 28, the guns switching positions and the fan speed.

By controlling the speed of the slide carriage, the amount of product projected into predetermined zones in the booth and therefore deposited, particularly onto areas of the client's body, by the guns 26 is determined. The guns are preset to deliver a known quantity of product per second. Therefore, if the slide carriage moves more quickly through its reciprocating stroke, or part of its stroke, then the area of the body which is to be sprayed at that part of the stroke will receive less product. This is because the guns pass more quickly over a particular part of the body when the slide carriage is moving more quickly, and *vice versa.*

The positions within the stroke of the slide carriage at which the guns are switched on and off, i.e, the times at which they are spraying and not spraying are also controlled by the control system.

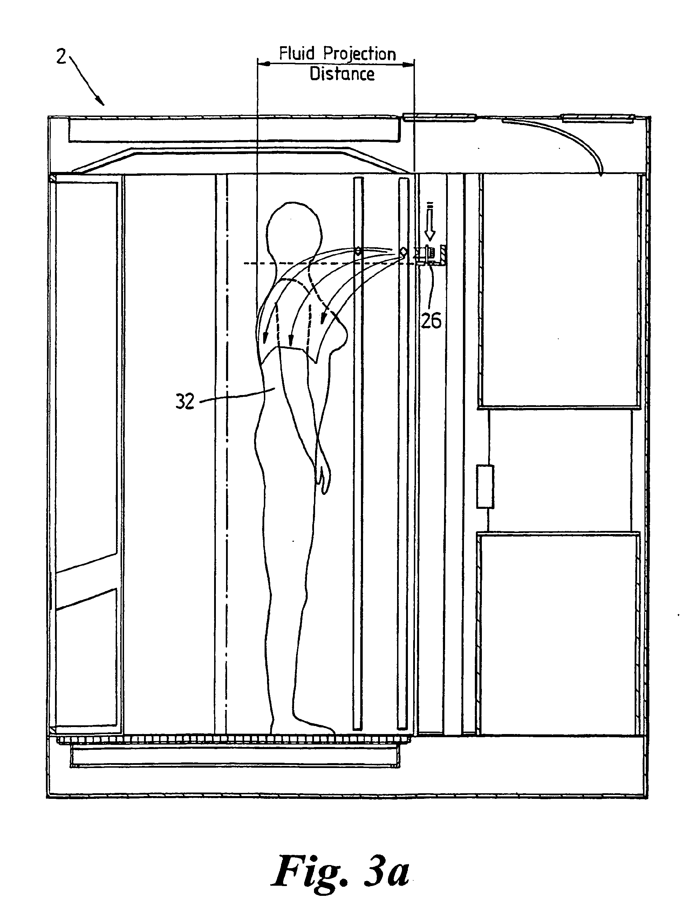

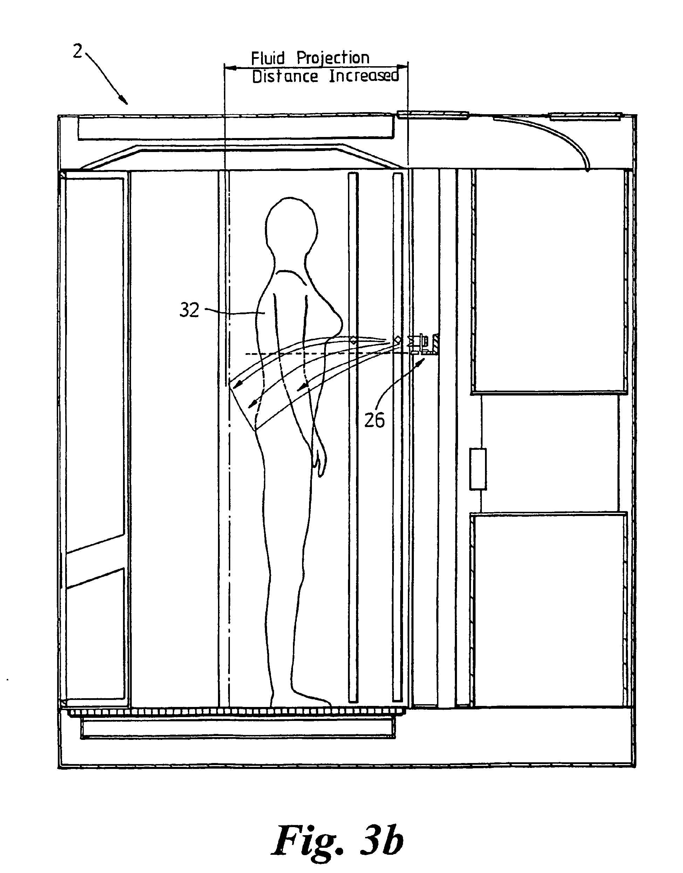

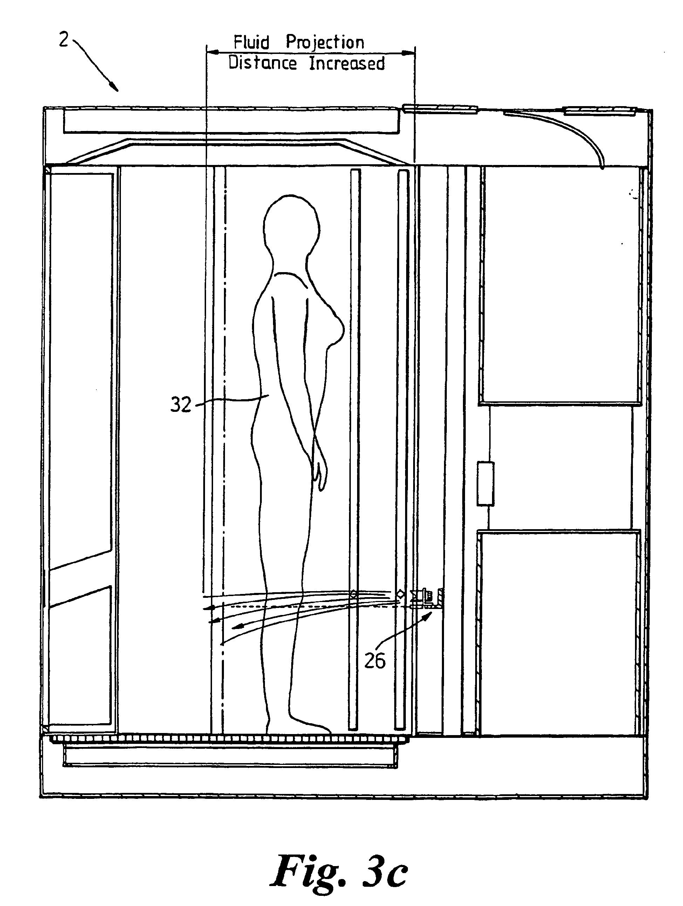

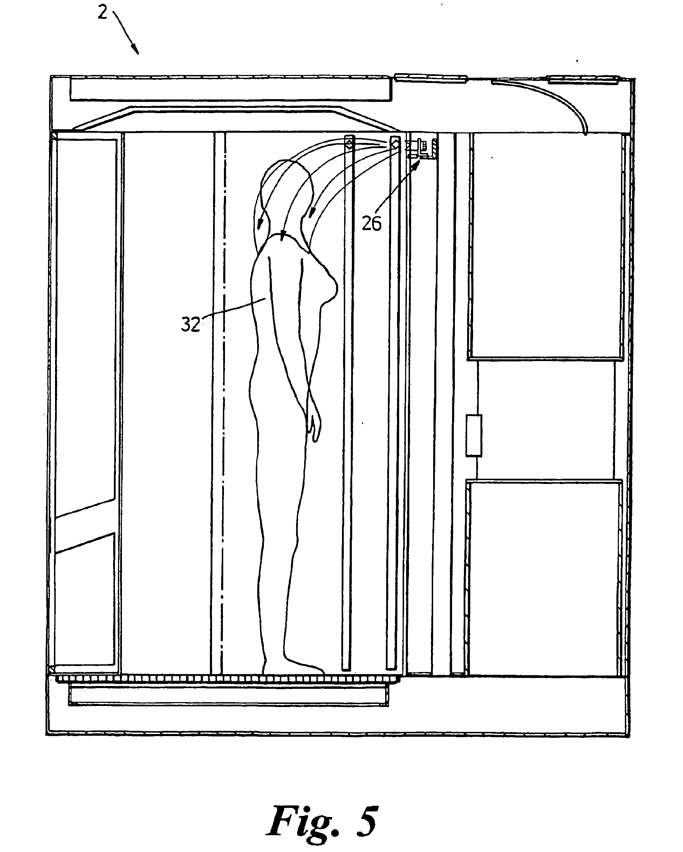

Controlling the speed of the fan 18 causes a greater or lesser volume of air to be moved per second through the finite volume of the booth 2. Therefore if the fan speed is increased, a greater volume of air will be recirculated per second within the booth. This causes an increase in the speed of downward extracting airflow within the spray chamber. This in turn increases the airflow speed within the spray chamber and causes the fluid projection distance to be decreased (see Figures 3a, 3b and 3c). By decreasing the fluid projection distance to an approximate equivalent to the distance between the client and the guns, it is possible to cause a greater deposition of product onto predetermined areas of the client, for example, onto the tops of the client's shoulders, therefore causing a higher degree of realism to the completed tan (see, for example, Figure 5). In addition, deposition of the product into difficult to reach areas such as the client's inner thighs can be enhanced by being able to increase the fluid projection distance at this point on the body.

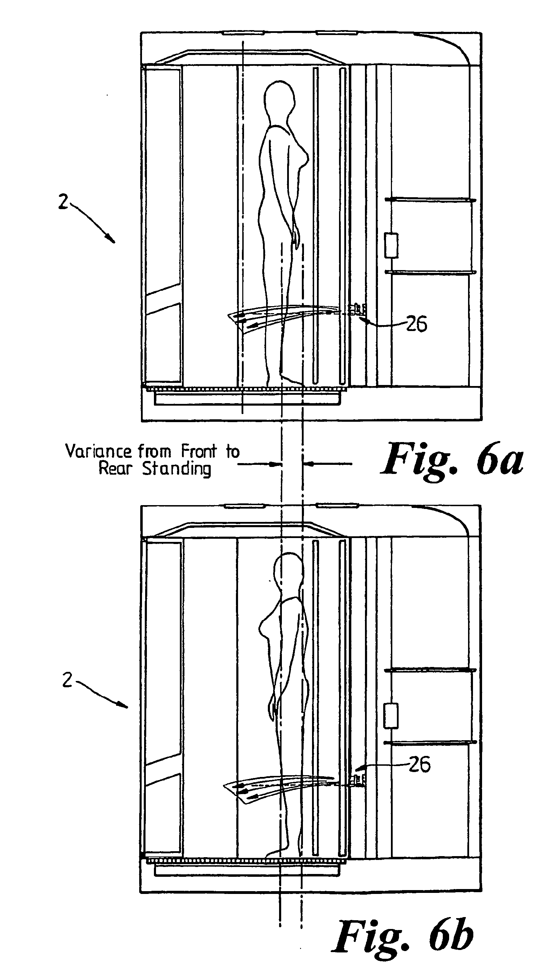

Control of the fan speed can also be beneficial in dealing with a problem caused by the client facing one way and then the other. The client is instructed to stand first facing one way and then to turn through approximately 180° to stand in an opposite direction. However, when the client presents the rear of her lower leg to the spray ring (as shown in Figures 6a and 6b), the lower leg is positioned closer to the spray ring than when she presents the front of her lower leg to the spray ring. This is due to the shape of the human foot, and where the lower leg intersects with the foot. By controlling the fluid projection it is possible to ensure that the coating is approximately identical on both the front lower leg and the back lower leg.

In an alternative embodiment, the spray ring is mounted for horizontal, as well as vertical movement. This means that the spray ring may be moved relative to the client in order to apply product to the back of the client. This obviates the need for the client to move through 180°.

In cases, where an operator of the booth will offer partial body tans to clients, it is commercially necessary to ensure that a client is not able to select a full body tan even though they have only paid for a partial body tan. In order to ensure that the client cannot select a full body tan when only paying for a partial body tan, the client can be issued with a small plug-in item. Within this item is an EPROM chip. This chip carries the necessary instruction to the control system to ensure that it carries out the relevant treatment. When inserted into the spray booth system, the EPROM chip carried in the device will ensure that the client will receive only the treatment that they have purchased. Once the plug in device is inserted, the control system will be alerted that the client is ready to proceed and will commence the body scan to determine the height of the client. If a client chooses to have a partial body tan, for example of her legs only, then the control system will calculate a position of the top of the client's legs and will begin the spraying process at this height.

Alternatively, data may be transmitted to the booth using radio frequency signals. This obviates the need to use an EPROM chip, which could be easily lost. An operator will select the treatment type purchased by the client at, for example, the front desk of the salon. A signal is then directed to the booth via a simple menu structure on the desk top controller. The booth sends a receipt signal to the controller upon receipt of its instructions, so that the operator knows that the booth has received the data and is ready to dispense the relevant treatment.

In this embodiment of the invention, the spray ring will continue to move along the entire stroke of the slide carriage, but the control system will activate the guns at the appropriate point to apply product to the legs only of the client.

However, in other embodiments, the spray ring may travel up to the appropriate point only and will not carry on to complete the entire stroke of the slide carriage.

Because the booth according to the present invention is able to detect the height of a client, the application of product is more efficient and there is less waste since an appropriate amount of product may be applied in accordance with the height of the client.

Further, in the case of shorter clients, the product will be sprayed at an appropriate height relative to the head of the client, which will reduce the possibility that the client will inhale the product. In prior art booths, where the height of the client cannot be taken into account, an unnecessarily long period of time may be spent on applying product to, and above, the client's face.

By means of the present invention, however, since it is possible to detect where the client's head is located, only sufficient product to apply the product to the face will be projected onto the face of the client, thus reducing the amount of product that the client could inhale.

Further, by means of the present invention, it is possible to apply different amounts of product to different parts of a client's body. The inventors have determined that certain areas of the body are capable of absorbing more product than others before excess application occurs. Excess application is undesirable as this causes the product to run off the skin leaving streaks in the finished application and therefore the tan. By being aware of the locations of various parts of the body that require differing amounts of product, and applying the product accordingly, the disadvantages of excess product application are minimised.

In prior art booths where it has not been possible to control the amount of product dispensed onto a person standing within the booth, the amount of product dispensed is limited by how much product can be absorbed by parts of the body that are less absorbent than others. This in turn limits the depth of the tan which can be produced by a sunless tanning product.

By means of the present invention, therefore, it is possible to maximise the amount of product applied to all body parts without the previously existing problems of over application.

With conventional booths used to apply sunless tanning product, if for example the amount of product applied to the body of a person standing in the booth was determined by the amount of product that could be absorbed by the calves of a person, then a light tan to the back would result.

By means of the present invention, it is possible to apply a maximum amount of product to each body part, by varying the amount of product applied to the different body parts.

It appears that an even sunless tan can be achieved, not from ensuring that the amount of product applied to a person's body is the same all over the body, but rather from ensuring that a maximum amount of product is applied to each particular body part, or a common percentage of this maximum amount is applied to all body parts. This results in an even tan being obtained, the strength which can be determined by the percentage of the maximum amount of product that is applied to particular body parts. The resulting tan is more realistic because certain parts of the body, for example, the shoulders can be given a darker tan which is consistent with the appearance of a natural tan.

By means of the present invention therefore a highly specific and controlled delivery of atomised products such as a sunless tanning product may be applied to the client. There is no need for the client to manually manipulate the product once it has been applied to the skin in order to ensure even coverage.

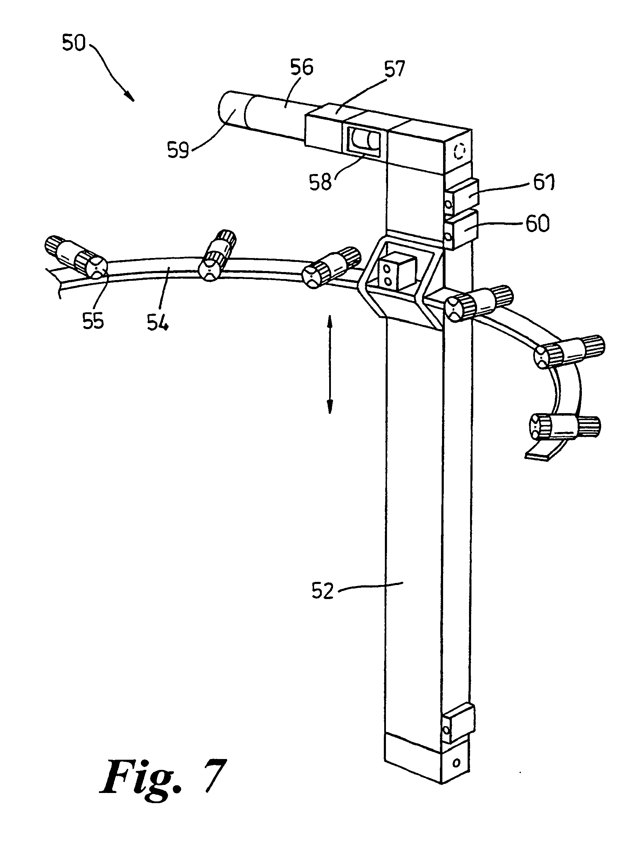

There is shown in Figure 7 details of a spray ring 50 suitable for use in booth 2.

The interior of booth 2 contains a slide unit 52 which supports a spray arm 54 to provide vertical upward and downward movement of arm 54 over the entire height of the booth. In other embodiments, there may be a plurality of slide units.

Spray arm 54 comprises a horizontal arcuate arm containing a plurality of spray guns 55 to direct tanning lotion inwardly and horizontally generally towards the centre of arm 54 and hence onto any occupant of the booth.

Thus, spray arm 54 can be shaped to form an arc of from 0° to 360° However, in this embodiment the arc as measured between the two outermost guns is 85°. The spray guns may extend along the entire periphery of the arm.

The spray guns are positioned so as to direct the lotion horizontally. In a variant, they may be positioned to direct the lotion at a small angle inclined to the horizontal. In another variant, the guns may be positioned to direct the lotion at various different angles either upwardly or downwardly, and either in a random manner or in an organised, progressively changing manner.

Spraying mechanism 50 also has a motor 57 and a gearbox 58 to provide the vertical motion of slide units 52, 53.

The "open" configuration of spray arm 54, has been developed with safety in mind. The spray arm 54 allows client access in from one side, regardless of the position within the spray chamber and it enables a quick exit in case emergency procedures need to be followed. It also allows for a quick exit if the client, for whatever reason, feels uncomfortable with the process. If movement of the spray arm 54 is halted at any position during its travel, the client is still able to exit the booth with minimal discomfort or danger.

In order to spray the client over the whole body using this design of spray arm 54, the application of tanning lotion must be applied in two steps. In the first step the front section of the body is sprayed; the client then turns through 180 degrees, and actuates operation of the spray arm 54 again, thereby applying lotion to the rear section of the body. Multiple spray guns would only be seen on one section the spray arm.

Alternative designs may allow for pneumatically actuated slide systems, but they tend to lack precise speed control, acceleration and deceleration parameters as compared to the programmable, electrically driven slide system.

The drive system is based on a servo motor 56 arrangement, allowing for the programmable switching of the spray guns according to the different requirements of the client. Alternative drive solutions include stepper motor control, AC and DC stepper motors.

The spray ring 50 further comprises a flexible coupling 58 which connects the servo motor to the slide carriage 52. The spray ring 50 also comprises an encoder for enabling the spray ring to be programmed by the control system.

The spray ring 50 further comprises a carriage home position sensor 60 which senses when the spray ring has reached its maximum height, and an over travel safety switch 61 which would cut off power to the spray ring in the event that it travelled beyond its maximum height.

In addition to the drive motor arrangement, the rate of rotation is reduced using a gearbox arrangement 57. This provides a higher torque, which is required to overcome the weight of the spray arm 54 and actually allows for more precise positioning of the slide systems.

In order to keep the doors closed, a magnetic latch system is used. This allows the doors to stay closed against the spring-loaded "opening" system, but also allows for manual release of the doors should an emergency procedure arise, requiring the client to exit the booth. If a power cut experienced, the doors are automatically opened by default, immediately providing a clear exit for the client.

Alternatively, a mechanical latch may be used to close the doors.

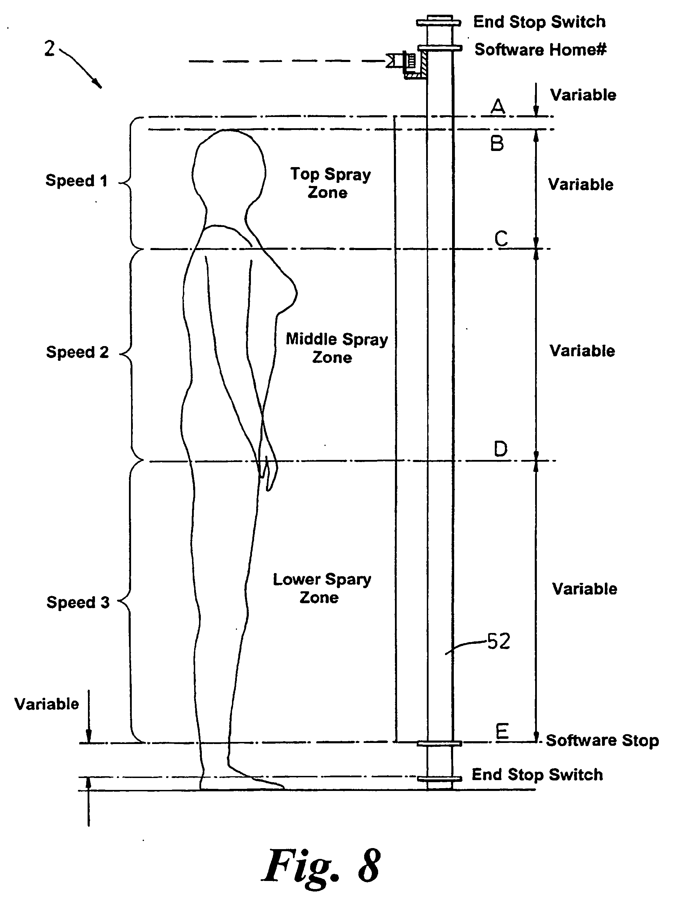

Turning now to Figure 8, the operation of a booth according to the present invention is described in more detail.

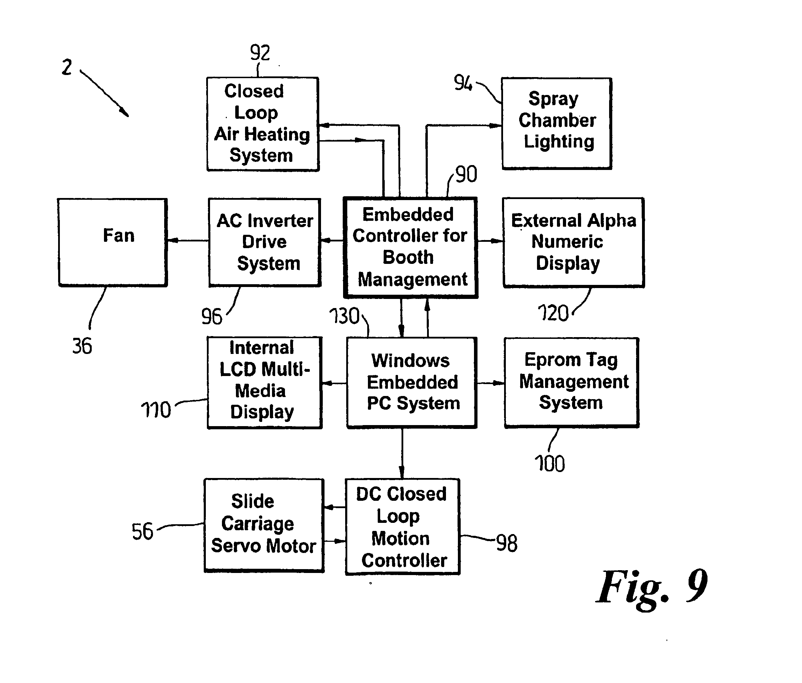

Set out below is process flow chart and sequence of operations describing the operation of the booth 2 with reference to Figure 8. 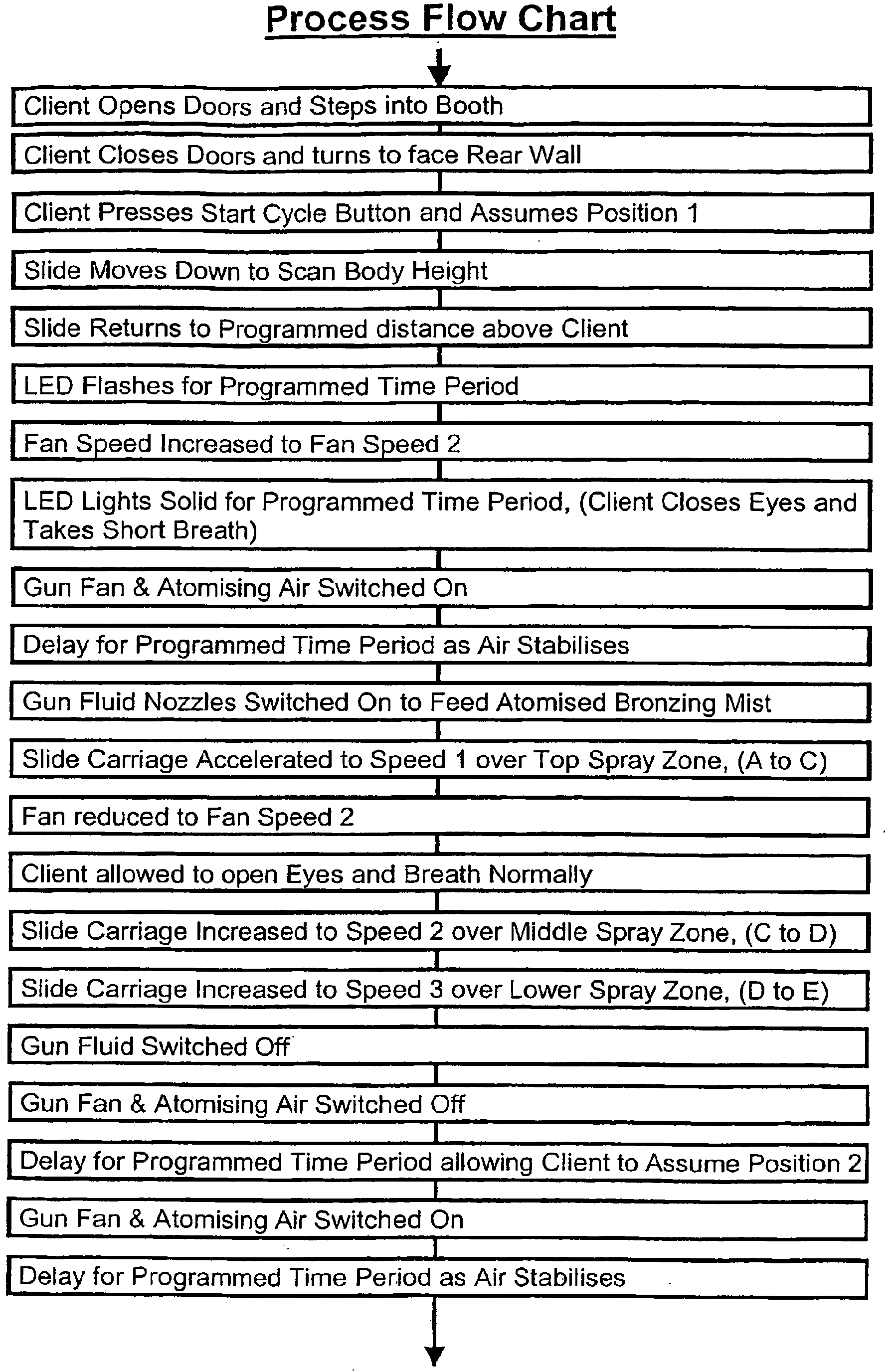 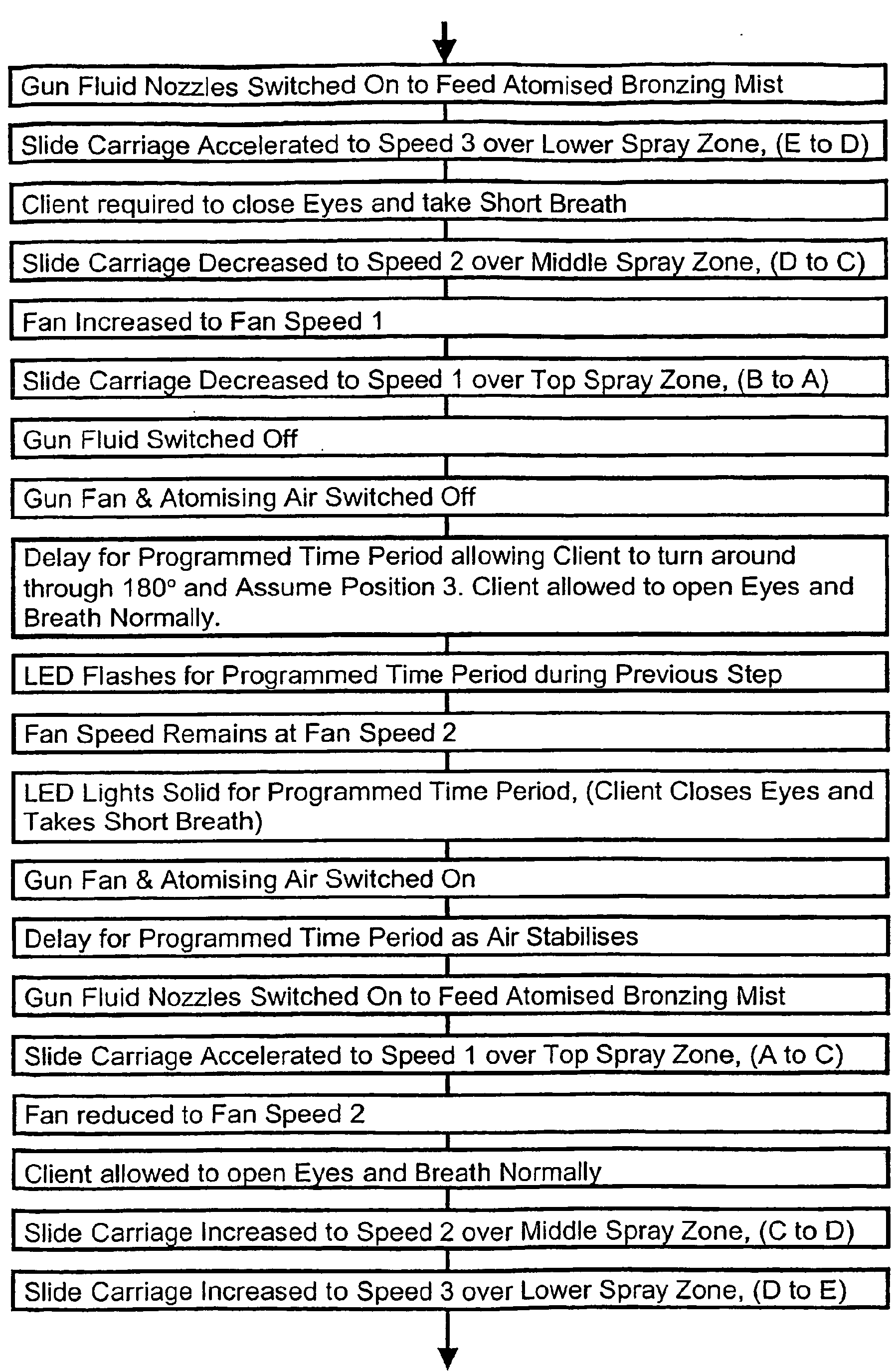 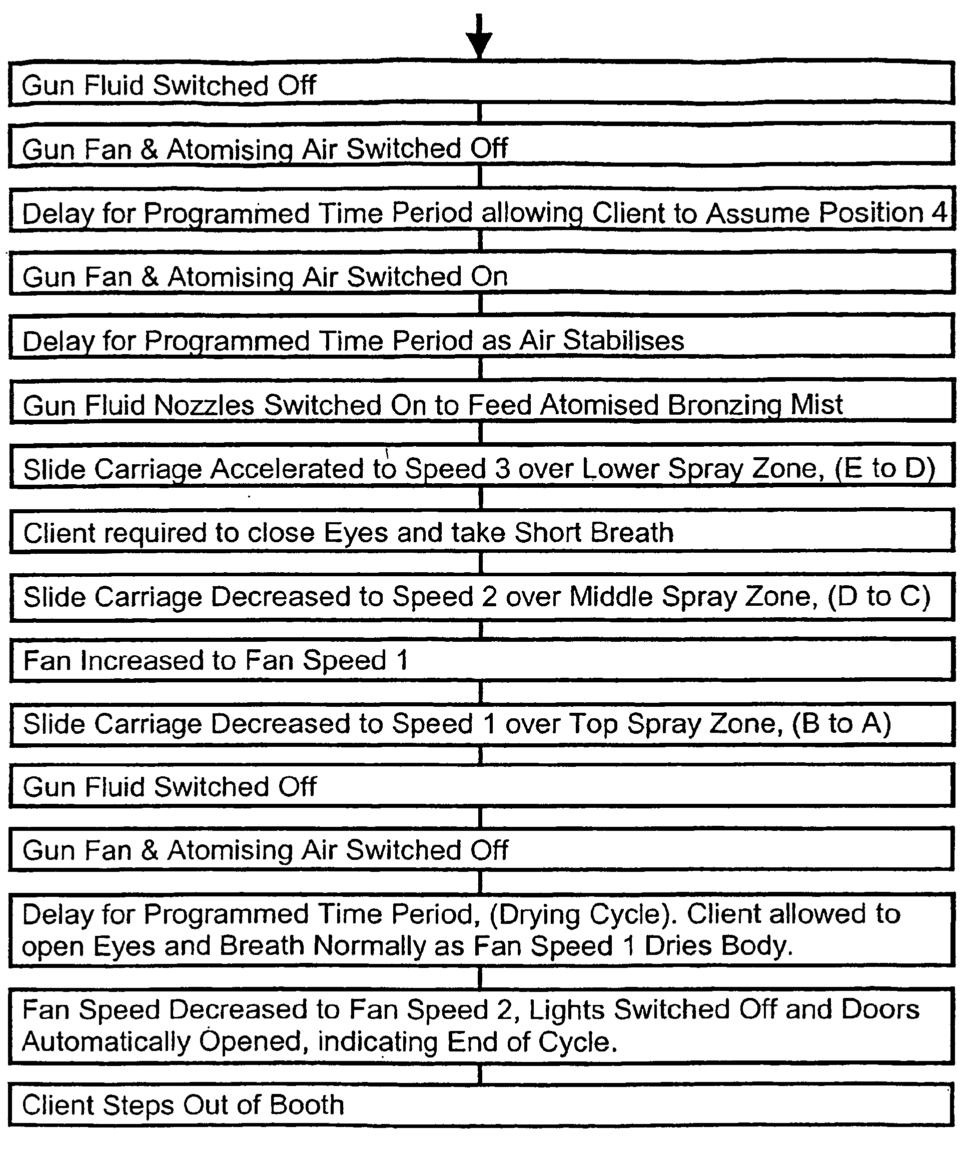 Turing now to Figure 9, a block diagram showing the components of the booth 2 is illustrated. The booth 2 comprises a control system 90 which controls the various components forming the booth 2. In particular, the booth 2 comprises a closed loop air heating system 92 and a spray chamber lighting system 94. An AC inverter drive system 96 is used to power the fan 36. Movement of the spray ring is controlled by the slide carriage servo motor 56 which in turn is controlled by a DC closed loop motion controller 98. If an EPROM chip is use, then an EPROM tag management system 100 is also controlled by the control system 90. The booth further comprises an internal LCD multi-media display 110 which allows the client positioned within the booth to see the settings of the booth 2, and an external alpha numeric display 120 which allows an operator to monitor the spraying operation. The control system 90 is run in conjunction with a Windows (RTM) embedded PC system 130.

In other embodiments, the LCD display 110 may be omitted. In such embodiments, there is no longer a requirement for a Windows (RTM) embedded PCT system 130.

## Claims

1. A booth (2) for accommodating a person, the booth defining a booth volume and comprising:
a base portion (6) and a top portion (8);
flow means (10,12) for causing a downward airflow within the booth;
projecting means (22) for projecting a sunless tanning product into at least some of the booth volume within the booth and, in use, onto a body of a person positioned in the booth; and
control means (90) for controlling the projection of the product,
**characterised in that** the control means further comprises a sensor (30) for sensing the height of the person, and estimating means for estimating one or more other parameters of the body based on the height, the control means controlling the amount of product directed to predetermined parts of the body in response to the height of the person.

2. A booth (2) according to Claim 1 wherein the sensor comprises an infra-red sensor.

3. A booth (2) according to any one of the preceding claims further comprising movement means (28) for effecting movement of the projecting means.

4. A booth (2) according to Claim 3 wherein the control means (90) is for controlling operation of the movement means.

5. A booth (2) according to Claim 3 or Claim 4 wherein the movement means (28) effects vertical movement of the projecting means.

6. A booth (2) according to Claim 5 wherein the movement means comprises at least one slider unit (28) moveable vertically between two positions, the slider unit supporting the projecting means.

7. A booth (2) according to any one of the preceding claims wherein the projecting means comprises at least one nozzle (26) to project the product.

8. A booth (2) according to any one of Claims 1 to 7 wherein the projecting means (22) comprises a plurality of nozzles adapted to project the product

9. A booth (2) according to Claim 5 or Claim 6 further comprising adjustment means for adjusting the attitude of the nozzles.

10. A booth (2) according to Claim 9 wherein the control means (90) is for controlling operation of the adjustment means.

11. A booth (2) according to any one of Claims 7 to 10 wherein the projection means (22) comprises a nozzle support (54) defining a substantially arcuate shape, the nozzles being positioned to spray the product into an area defined by the nozzle support.

12. A booth (2) according to any one of the preceding claims wherein the projecting means (22) comprises one or more spray guns (26;55) which are directed to spray product horizontally and/or at an angle to the horizontal (whether upwardly or downwardly) and/or some combination of these.

13. A booth (2) according to any one of the preceding claims wherein the projecting means comprises one or more slider units (52,53) supporting an arcuate spray arm (54) comprising a plurality of spray guns (55).

14. A booth (2) according to any one of the preceding claims further comprising a fluid delivery system for delivering the product into the booth via the projecting means.

15. A booth (2) according to Claim 14 wherein the booth delivery system forms part of a recirculating fluid circuit which includes the projecting means.

16. A booth (2) according to Claims 14 or Claim 15 wherein the fluid is contained within a fluid container.

17. A booth (2) according to any one of Claims 14 to 16 wherein the fluid delivery system is adapted to deliver air to the projection means at a predetermined air pressure.

18. A booth (2) according to Claim 17 wherein the predetermined air pressure is variable.

19. A booth (2) according to any one of Claims 14 to 18 wherein the fluid delivery system is adapted to deliver product in the form of a fluid to the projection means.

20. A booth (2) according to Claim 19 wherein the fluid delivery system is adapted to deliver product to the projection means at a predetermined pressure.

21. A booth (2) according to Claim 20 wherein the predetermined pressure is variable.

22. A booth (2) according to any one of Claims 14 to 21 wherein the control means (90) is adapted to control operation of the fluid delivery system.

23. A booth (2) according to any one of Claims 14 to 22 further comprising pressure means for controlling the fluid pressure.

24. A booth according to Claim 23 wherein the pressure means comprises a fluid pump.

25. A booth (2) according to many one of the preceding claims further comprising recirculating means for recirculating the air within the booth.

26. A booth (2) according to Claims 25 wherein the control means (90) is for controlling operation of the recirculating means.

27. A booth (2) according to Claim 24 or Claim 25 wherein the recirculating means comprises a fan (18).

28. A booth (2) according to any one of the preceding claims further comprising a filter.

29. A booth (2) according to Claim 28 wherein the filter comprises a plurality of filters forming a filtration system (20).

30. A booth (2) according to any one of the preceding claims wherein the flow means comprises a first plenum (10) of positive pressure located in the top portion of the booth, and a second plenum (12) of negative pressure located in the base of the booth.

31. A booth (2) according to any one of the preceding claims wherein the downward air flow occupies a predetermined volume (4) within the booth.

32. A booth (2) according to Claim 31 wherein the predetermined volume (4) comprises a portion of the booth volume.

33. A booth (2) according to any one of the preceding claims further comprising temperature means (34) for controlling the temperature of the air.

34. A booth (2) according to Claim 33 wherein the temperature means (34) is adapted to heat the air.

35. A booth (2) according to Claim 33 wherein the temperature means is adapted to cool the air.

36. A booth (2) according to any one of Claims 30 to 35 wherein the first and second plenums are connected to a duct (16), and the air recirculating means is located within the duct.

37. A booth (2) according to any one of the preceding claims wherein the projecting means comprises a remotely operable tooL

38. A booth (2) according to any one of the preceding claims wherein the control means (90) is operable on the projecting means to project specified amounts of the product in selected regions of the booth volume, the specified amounts varying from zero to maximum flow of the product from the projecting means.

39. A booth (2) according to Claim 38 whereon the projecting means is movable along a path within the booth, and the control system (90) is adapted to operate the projecting means as it moves along the path in accordance with predetermined instructions.

40. A method of applying a sunless tanning product to a human body using a boot (2), the booth defining a booth volume (4) and comprising:
a base portion (6) and a top portion (8);
flow means (10,12) for causing a downward air flow within the booth;
projecting means (22) for projecting a product into at least some of the booth volume within the booth and, in use, onto a body of a person positioned in the booth; and
control means (90) for controlling the projection of the product the method comprising the steps of:
sensing the height of a human body accommodated in the booth;
estimating one or more other parameters of the body based on the height;
projecting a product into the booth and therefore onto the body;
**characterised in that** the method comprises the further step of controlling the projection of product into predetermined zones in the booth in accordance with the height of the body.

41. A method according to Claim 40 wherein the step of projecting a product into the booth comprises spraying the product via the projecting means into the booth.

42. A method according to Claim 40 or Claim 41 wherein the booth further comprises a fluid delivery system for delivering air and product to the projection means, and the step of controlling the projection of product into predetermined zones within the booth in accordance with the height of the body comprises one or more of the following steps:
controlling the pressure at which product is delivered to the projecting means;
controlling the pressure at which air is delivered to the projecting means.

43. A method according to any one of Claims 40 to 42 wherein the step of controlling the amount of product projected into predetermined zones further comprises the step of causing the projecting means to move vertically within the booth, and controlling the speed at which the projecting means move.

44. A method according to any one of Claims 40 to 43 wherein the step of controlling the amount of product projected further comprises the step of controlling the air speed of the downward air flow within the booth.

## Patentansprüche

1. Kabine (2) zum Aufnehmen einer Person, wobei die Kabine ein Kabinenvolumen definiert und Folgendes umfasst:
einen Basisabschnitt (6) und einen Dachabschnitt (8);
ein Strömungsmittel (10, 12) zum Erzeugen eines abwärts gerichteten Luftstroms innerhalb der Kabine;
ein Ausspritzmittel (22) zum Ausspritzen eines Selbstbräunungsprodukts in mindestens einen Teil des Kabinenvolumens innerhalb der Kabine und, während des Gebrauchs, auf einen Körper einer Person, die sich in der Kabine befindet; und
ein Steuerungsmittel (90) zum Steuern des Ausspritzens des Produkts,
**dadurch gekennzeichnet, dass** das Steuerungsmittel des Weiteren einen Sensor (30) zum Detektieren der Größe der Person und ein Schätzmittel zum Schätzen eines oder mehrerer weiterer Parameter des Körpers auf der Grundlage der Körpergröße umfasst, wobei das Steuerungsmittel die Menge des Produkts, das auf festgelegte Körperregionen gerichtet wird, in Reaktion auf die Größe der Person steuert.

2. Kabine (2) nach Anspruch 1, wobei der Sensor einen Infrarotsensor umfasst.

3. Kabine (2) nach einem der vorangehenden Ansprüche, die des Weiteren ein Bewegungsmittel (28) zum Bewirken einer Bewegung des Ausspritzmittels umfasst.

4. Kabine (2) nach Anspruch 3, wobei das Steuerungsmittel (90) zum Steuern der Funktion des Bewegungsmittels dient.

5. Kabine (2) nach Anspruch 3 oder Anspruch 4, wobei das Bewegungsmittel (28) eine vertikale Bewegung des Ausspritzmittels veranlasst.

6. Kabine (2) nach Anspruch 5, wobei das Bewegungsmittel mindestens eine Schiebereinheit (28) umfasst, die vertikal zwischen zwei Positionen bewegt werden kann, wobei die Schiebereinheit das Ausspritzmittel trägt.

7. Kabine (2) nach einem der vorangehenden Ansprüche, wobei das Ausspritzmittel mindestens eine Düse (26) zum Ausspritzen des Produkts umfasst.

8. Kabine (2) nach einem der Ansprüche 1 bis 7, wobei das Ausspritzmittel (22) mehrere Düsen umfasst, die zum Ausspritzen des Produkts ausgelegt sind.

9. Kabine (2) nach Anspruch 5 oder Anspruch 6, die des Weiteren ein Justiermittel zum Justieren der Düsenstellung umfasst.

10. Kabine (2) nach Anspruch 9, wobei das Steuerungsmittel (90) zum Steuern der Funktion des Justiermittels dient.

11. Kabine (2) nach einem der Ansprüche 7 bis 10, wobei das Ausspritzmittel (22) einen Düsenträger (54) umfasst, der eine im Wesentlichen bogenförmige Gestalt definiert, wobei die Düsen so positioniert sind, dass sie das Produkt in einen durch den Düsenträger definierten Bereich sprühen.

12. Kabine (2) nach einem der vorangehenden Ansprüche, wobei das Ausspritzmittel (22) eine oder mehrere Sprühpistolen (26, 55) umfasst, die so ausgerichtet sind, dass sie das Produkt horizontal und/oder in einem Winkel zur Horizontalen (aufwärts oder abwärts) und/oder in einer Kombination dieser Richtungen versprühen.

13. Kabine (2) nach einem der vorangehenden Ansprüche, wobei das Ausspritzmittel eine oder mehrere Schiebereinheiten (52, 53) umfasst, die einen bogenförmigen Sprüharm (54) tragen, der mehrere Sprühpistole (55) umfasst.

14. Kabine (2) nach einem der vorangehenden Ansprüche, die des Weiteren ein Fluidzufuhrsystem zum Ausgeben des Produkts in die Kabine über das Ausspritzmittel umfasst.

15. Kabine (2) nach Anspruch 14, wobei das Kabinenzufuhrsystem einen Teil eines Fluidumlaufkreises bildet, der das Ausspritzmittel enthält.

16. Kabine (2) nach Anspruch 14 oder Anspruch 15, wobei das Fluid in einem Fluidbehälter gespeichert ist.

17. Kabine (2) nach einem der Ansprüche 14 bis 16, wobei das Fluidzufuhrsystem dafür ausgelegt ist, Luft mit einem vorgegebenen Luftdruck zu dem Ausspritzmittel zu leiten.

18. Kabine (2) nach Anspruch 17, wobei der vorgegebene Luftdruck variabel ist.

19. Kabine (2) nach einem der Ansprüche 14 bis 18, wobei das Fluidzufuhrsystem dafür ausgelegt ist, das Produkt in Form eines Fluids zu dem Ausspritzmittel zu leiten.

20. Kabine (2) nach Anspruch 19, wobei das Fluidzufuhrsystem dafür ausgelegt ist, das Produkt mit einem vorgegebenen Druck zu dem Ausspritzmittel zu leiten.

21. Kabine (2) nach Anspruch 20, wobei der vorgegebene Druck variabel ist.

22. Kabine (2) nach einem der Ansprüche 14 bis 21, wobei das Steuerungsmittel (90) dafür ausgelegt ist, die Funktion des Fluidzufuhrsystems zu steuern.

23. Kabine (2) nach einem der Ansprüche 14 bis 22, die des Weiteren ein Druckmittel zum Steuern des Fluiddrucks umfasst.

24. Kabine nach Anspruch 23, wobei das Druckmittel eine Fluidpumpe umfasst.

25. Kabine (2) nach einem der vorangehenden Ansprüche, die des Weiteren ein Umluftmittel zum Rezirkulieren der Luft innerhalb der Kabine umfasst.

26. Kabine (2) nach Anspruch 25, wobei das Steuerungsmittel (90) zum Steuern der Funktion des Umluftmittels dient.

27. Kabine (2) nach Anspruch 24 oder Anspruch 25, wobei das Umluftmittel ein Gebläse (18) umfasst.

28. Kabine (2) nach einem der vorangehenden Ansprüche, die des Weiteren einen Filter umfasst.

29. Kabine (2) nach Anspruch 28, wobei das Filter mehrere Filter umfasst, die ein Filtrationssystem (20) bilden.

30. Kabine (2) nach einem der vorangehenden Ansprüche, wobei das Strömungsmittel eine erste Sammelkammer (10) mit positivem Druck umfasst, die in dem Dachabschnitt der Kabine angeordnet ist, und eine zweite Sammelkammer (12) mit negativem Druck umfasst, die in der Basis der Kabine angeordnet ist.

31. Kabine (2) nach einem der vorangehenden Ansprüche, wobei der abwärts gerichtete Luftstrom ein vorgegebenes Volumen (4) innerhalb der Kabine einnimmt.

32. Kabine (2) nach Anspruch 31, wobei das vorgegebene Volumen (4) einen Teil des Kabinenvolumens umfasst.

33. Kabine (2) nach einem der vorangehenden Ansprüche, die des Weiteren ein Temperaturmittel (34) zum Steuern der Temperatur der Luft umfasst.

34. Kabine (2) nach Anspruch 33, wobei das Temperaturmittel (34) dafür ausgelegt ist, die Luft zu erwärmen.

35. Kabine (2) nach Anspruch 33, wobei das Temperaturmittel dafür ausgelegt ist, die Luft zu kühlen.

36. Kabine (2) nach einem der Ansprüche 30 bis 35, wobei die erste und die zweite Sammelkammer mit einem Kanal (16) verbunden sind und das Umluftmittel innerhalb des Kanals angeordnet ist.

37. Kabine (2) nach einem der vorangehenden Ansprüche, wobei das Ausspritzmittel ein fernbedienbares Werkzeug umfasst.

38. Kabine (2) nach einem der vorangehenden Ansprüche, wobei das Steuerungsmittel (90) das Ausspritzmittel veranlasst, bestimmte Mengen des Produkts in ausgewählte Regionen des Kabinenvolumens auszuspritzen, wobei die bestimmten Mengen von null bis zu einem maximalen Fluss des Produkts aus dem Ausspritzmittel reichen.

39. Kabine (2) nach Anspruch 38, wobei das Ausspritzmittel entlang eines Pfades innerhalb der Kabine bewegt werden kann, und das Steuerungssystem (90) dafür ausgelegt ist, das Ausspritzmittel gemäß vorgegebenen Instruktionen zu betätigen, während es sich entlang des Pfades bewegt.

40. Verfahren zum Auftragen eines Selbstbräunungsprodukts auf den Körper einer Person mit Hilfe einer Kabine (2), wobei die Kabine ein Kabinenvolumen (4) definiert und Folgendes umfasst:
einen Basisabschnitt (6) und einen Dachabschnitt (8);
ein Strömungsmittel (10, 12) zum Erzeugen eines abwärts gerichteten Luftstromes innerhalb der Kabine;
ein Ausspritzmittel (22) zum Ausspritzen eines Produkts in mindestens einen Teil des Kabinenvolumens innerhalb der Kabine und, während des Gebrauchs, auf einen Körper einer Person, die sich in der Kabine befindet; und
ein Steuerungsmittel (90) zum Steuern des Ausspritzens des Produkts, wobei das Verfahren folgende Schritte umfasst:
Detektieren der Größe einer in der Kabine befindlichen Person;
Schätzen eines oder mehrerer weiterer Parameter des Körpers anhand der Körpergröße;
Ausspritzen eines Produkts in die Kabine und dabei auf den Körper;
**dadurch gekennzeichnet, dass** das Verfahren den weiteren Schritt umfasst, das Ausspritzen des Produkts in vorgegebene Zonen in der Kabine gemäß der Körpergröße zu steuern.

41. Verfahren nach Anspruch 40, wobei der Schritt des Ausspritzens eines Produkts in die Kabine das Sprühen des Produkts über das Ausspritzmittel in die Kabine umfasst.

42. Verfahren nach Anspruch 40 oder Anspruch 41, wobei die Kabine des Weiteren ein Fluidzufuhrsystem zum Zuführen von Luft und Produkt zu dem Ausspritzmittel umfasst, und der Schritt des Steuerns des Ausspritzens des Produkts in vorgegebene Zonen innerhalb der Kabine gemäß der Körpergröße einen oder mehrere der folgenden Schritte umfasst:
Steuern des Drucks, mit dem das Produkt zum Ausspritzmittel geleitet wird;
Steuern des Drucks, mit dem Luft zu dem Ausspritzmittel geleitet wird.

43. Verfahren nach einem der Ansprüche 40 bis 42, wobei der Schritt des Steuerns der Menge an Produkt, das in vorgegebene Zonen ausgespritzt wird, des Weiteren den Schritt umfasst, das Ausspritzmittel zu einer vertikalen Bewegung innerhalb der Kabine zu veranlassen und die Bewegungsgeschwindigkeit des Ausspritzmittels zu steuern.

44. Verfahren nach einem der Ansprüche 40 bis 43, wobei der Schritt des Steuerns der Menge des ausgespritzten Produkts des Weiteren den Schritt umfasst, die Luftgeschwindigkeit des abwärts gerichteten Luftstroms innerhalb der Kabine zu steuern.

## Revendications

1. Cabine pour accueillir une personne, la cabine définissant un volume de cabine et comprenant:
une partie de base (6) et une partie supérieure (8);
des moyens d'écoulement (10, 12) pour provoquer un écoulement d'air descendant à l'intérieur de la cabine;
des moyens de projection (22) pour projeter un produit de bronzage sans soleil dans au moins une partie du volume de cabine à l'intérieur de la cabine et à l'usage, sur un corps d'une personne positionnée dans la cabine; et
des moyens de commande (90) pour commander la projection du produit,
**caractérisée en ce que** les moyens de commande comprennent en outre un capteur (30) pour détecter la stature de la personne et des moyens d'estimation pour estimer un ou plusieurs autres paramètres du corps en fonction de la stature, les moyens de commande commandant la quantité de produit dirigé sur des parties prédéterminées du corps en réponse à la stature de la personne.

2. Cabine (2) selon la revendication 1,- dans laquelle le capteur comprend un capteur infrarouge.

3. Cabine (2) selon l'une quelconque des revendications précédentes, comprenant en outre des moyens de mouvement (28) pour effectuer un mouvement des moyens de projection.

4. Cabine (2) selon la revendication 3, dans laquelle les moyens de commande (90) sont prévus pour commander le fonctionnement des moyens de mouvement.

5. Cabine (2) selon la revendication 3 ou la revendication 4, dans laquelle les moyens de mouvement (28) effectuent un mouvement vertical des moyens de projection.

6. Cabine (2) selon la revendication 5, dans laquelle les moyens de mouvement comprennent au moins une unité de glissière (28) verticalement mobile entre deux positions, l'unité de glissière supportant les moyens de projection.

7. Cabine (2) selon l'une quelconque des revendications précédentes, dans laquelle les moyens de projection comprennent au moins une buse (26) pour projeter le produit.

8. Cabine (2) selon l'une quelconque des revendications 1 à 7, dans laquelle les moyens de projection (22) comprennent une pluralité de buses adaptées pour projeter le produit.

9. Cabine (2) selon la revendication 5 ou la revendication 6, comprenant en outre des moyens d'ajustement pour ajuster l'attitude des buses.

10. Cabine (2) selon la revendication 9, dans laquelle les moyens de commande (90) sont prévus pour commander le fonctionnement des moyens d'ajustement.

11. Cabine (2) selon l'une quelconque des revendications 7 à 10, dans laquelle les moyens de projection (22) comprennent un support de buse (54) définissant une forme sensiblement arquée, les buses étant positionnées pour pulvériser le produit dans une zone définie par le support de buse.

12. Cabine (2) selon l'une quelconque des revendications précédentes, dans laquelle les moyens de projection (22) comprennent un ou plusieurs pistolets de pulvérisation (26 ; 55) qui sont dirigés pour pulvériser le produit horizontalement et/ou selon un angle par rapport à l'horizontale (vers le haut ou vers le bas) et/ou une certaine combinaison de ces derniers.

13. Cabine (2) selon l'une quelconque des revendications précédentes, dans laquelle les moyens de projection comprennent une ou plusieurs unités de glissière (52, 53) supportant un bras de pulvérisation arqué (54) comprenant une pluralité de pistolets de pulvérisation (55).

14. Cabine (2) selon l'une quelconque des revendications précédentes, comprenant en outre un système de distribution de fluide pour distribuer le produit dans la cabine via les moyens de projection.

15. Cabine (2) selon la revendication 14, dans laquelle le système de distribution de cabine fait partie d'un circuit de fluide de recirculation qui comprend les moyens de projection.

16. Cabine (2) selon la revendication 14 ou la revendication 15, dans laquelle le fluide est contenu à l'intérieur d'un récipient de fluide.

17. Cabine (2) selon l'une quelconque des revendications 14 à 16, dans laquelle le système de distribution de fluide est adapté pour distribuer de l'air aux moyens de projection à une pression d'air prédéterminée.

18. Cabine (2) selon la revendication 17, dans laquelle la pression d'air prédéterminée est variable.

19. Cabine (2) selon l'une quelconque des revendications 14 à 18, dans laquelle le système de distribution de fluide est adapté pour distribuer le produit sous la forme d'un fluide aux moyens de projection.

20. Cabine (2) selon la revendication 19, dans laquelle le système de distribution de fluide est adapté pour distribuer le produit aux moyens de projection à une pression prédéterminée.

21. Cabine (2) selon la revendication 20, dans laquelle la pression prédéterminée est variable.

22. Cabine (2) selon l'une quelconque des revendications 14 à 21, dans laquelle les moyens de commande (90) sont adaptés pour commander le fonctionnement du système de distribution de fluide.

23. Cabine (2) selon l'une quelconque des revendications 14 à 22, comprenant en outre des moyens de pression pour commander la pression de fluide.

24. Cabine (2) selon la revendication 23, dans laquelle les moyens de pression comprennent une pompe de fluide.

25. Cabine (2) selon l'une quelconque des revendications précédentes comprenant en outre des moyens de recirculation pour faire recirculer l'air à l'intérieur de la cabine.

26. Cabine (2) selon la revendication 25, dans laquelle les moyens de commande (90) sont prévus pour commander le fonctionnement des moyens de recirculation.

27. Cabine (2) selon la revendication 24 ou la revendication 25, dans laquelle les moyens de recirculation comprennent un ventilateur (18).

28. Cabine (2) selon l'une quelconque des revendications précédentes, comprenant en outre un filtre.

29. Cabine (2) selon la revendication 28, dans laquelle le filtre comprend une pluralité de filtres formant un système de filtration (20).

30. Cabine (2) selon l'une quelconque des revendications précédentes, dans laquelle les moyens d'écoulement comprennent un premier plenum (10) de pression positive situé dans la partie supérieure de la cabine et un deuxième plenum (12) de pression négative situé dans la base de la cabine.

31. Cabine (2) selon l'une quelconque des revendications précédentes, dans laquelle l'écoulement d'air descendant occupe un volume prédéterminé (4) à l'intérieur de la cabine.

32. Cabine (2) selon la revendication 31, dans laquelle le volume prédéterminé (4) comprend une partie du volume de la cabine.

33. Cabine (2) selon l'une quelconque des revendications précédentes, comprenant en outre des moyens de température (34) pour contrôler la température de l'air.

34. Cabine (2) selon la revendication 33, dans laquelle les moyens de température (34) sont adaptés pour chauffer l'air.

35. Cabine (2) selon la revendication 33, dans laquelle les moyens de température sont adaptés pour refroidir l'air.

36. Cabine (2) selon l'une quelconque des revendications 30 à 35, dans laquelle les premier et deuxième plenums sont raccordés à un conduit (16) et les moyens de recirculation d'air sont positionnés à l'intérieur du conduit.

37. Cabine (2) selon l'une quelconque des revendications précédentes, dans laquelle les moyens de projection comprennent un outil pouvant être actionné à distance.

38. Cabine (2) selon l'une quelconque des revendications précédentes, dans laquelle les moyens de commande (90) ont une action sur les moyens de projection pour projeter des quantités prédéterminées de produit dans des régions sélectionnées du volume de la cabine, les quantités spécifiées allant de zéro à l'écoulement maximum du produit par les moyens de projection.

39. Cabine (2) selon la revendication 38, dans laquelle les moyens de projection sont mobiles le long d'une trajectoire à l'intérieur de la cabine et le système de commande (90) est adapté pour faire fonctionner les moyens de projection lorsqu'ils se déplacent le long de la trajectoire selon des instructions prédéterminées.

40. Procédé pour appliquer un produit de bronzage sans soleil sur un corps humain en utilisant une cabine (2), la cabine définissant un volume de cabine (4) et comprenant :
une partie de base (6) et une partie supérieure (8) ;
des moyens d'écoulement (10, 12) pour provoquer un écoulement d'air descendant à l'intérieur de la cabine ;
des moyens de projection (22) pour projeter un produit dans au moins une certaine partie du volume de la cabine à l'intérieur de la cabine et à l'usage, sur un corps d'une personne positionnée dans la cabine ; et
des moyens de commande (90) pour commander la projection du produit, le procédé comprenant les étapes consistant à :
détecter la stature d'un corps humain logé dans la cabine ;
estimer un ou plusieurs autres paramètres du corps en fonction de la stature ;
projeter un produit dans la cabine et par conséquent sur le corps ;
**caractérisé en ce que** le procédé comprend l'étape supplémentaire consistant à contrôler la projection du produit dans des zones prédéterminées dans la cabine selon la stature du corps.

41. Procédé selon la revendication 40, dans lequel l'étape consistant à projeter un produit dans la cabine comprend l'étape consistant à pulvériser le produit via les moyens de projection dans la cabine.

42. Procédé selon la revendication 40 ou la revendication 41, dans lequel la cabine comprend en outre un système de distribution de fluide pour distribuer l'air et le produit aux moyens de projection et l'étape consistant à contrôler la projection du produit dans des zones prédéterminées à l'intérieur de la cabine selon la stature du corps comprend une ou plusieurs des étapes suivantes consistant à :
contrôler la pression à laquelle le produit est distribué aux moyens de projection ;
contrôler la pression à laquelle l'air est distribué aux moyens de projection.

43. Procédé selon l'une quelconque des revendications 40 à 42, dans lequel l'étape consistant à contrôler la quantité de produit projeté dans les zones prédéterminées comprend en outre les étapes consistant à amener les moyens de projection à se déplacer verticalement à l'intérieur de la cabine, et contrôler la vitesse à laquelle les moyens de projection se déplacent.

44. Procédé selon l'une quelconque des revendications 40 à 43, dans lequel l'étape consistant à contrôler la quantité de produit projeté comprend en outre l'étape consistant à contrôler la vitesse de l'air de l'écoulement d'air descendant à l'intérieur de la cabine.
